(12) **EUROPEAN PATENT SPECIFICATION**

(45) Date of publication and mention
of the grant of the patent:
**09.10.2002 Bulletin 2002/41**

(51) Int Cl.[7]: **A61K 38/44**, A01N 63/00,
A61P 31/04

(21) Application number: **94914718.5**

(22) Date of filing: **21.03.1994**

(86) International application number:
**PCT/US94/03089**

(87) International publication number:
**WO 94/023742 (27.10.1994 Gazette 1994/24)**

(54) **METHODS AND COMPOSITIONS FOR THE PREVENTION OR TREATMENT OF SEXUALLY TRANSMITTED DISEASES**

METHODEN UND ZUSAMMENSETZUNGEN ZUR VERHÜTUNG ODER BEHANDLUNG VON SEXUELL ÜBERTRAGBAREN KRANKHEITEN

PROCEDES ET COMPOSITIONS PROPHYLACTIQUES OU DE TRAITEMENT DES MALADIES TRANSMISES PAR VOIE SEXUELLE

(84) Designated Contracting States:
**AT BE CH DE DK ES FR GB GR IE IT LI LU MC NL PT SE**
Designated Extension States:
**SI**

(30) Priority: **15.04.1993 US 48647**

(43) Date of publication of application:
**31.01.1996 Bulletin 1996/05**

(73) Proprietor: **ExOxEmis, Inc.**
**Little Rock, AR 72201 (US)**

(72) Inventor: **ALLEN, Robert C.**
**San Antonio, TX 78209 (US)**

(74) Representative: **Spall, Christopher John**
**Barker Brettell,**
**138 Hagley Road**
**Edgbaston, Birmingham B16 9PW (GB)**

(56) References cited:
**EP-A- 0 361 908          EP-A- 0 500 387**

- **CHEMICAL ABSTRACTS, vol. 103, no. 17, 28 October 1985, Columbus, Ohio, US; abstract no. 136519d, T. AGETA ET AL. 'DETERMINATION OF FREE CHOLINE IN HUMAN SEMEN USING AN ISOTACHOPHORETIC ANALYZER.' page 233 ; & J. CHROMATOGR. vol. 343, no. 1 , 1985 pages 186 - 189**
- **JOURNAL OF EXPERIMENTAL MEDICINE vol. 174, no. 1 , July 1991 , NEW YORK, N.Y., US pages 289 - 292 S.J. KLEBANOFF ET AL. 'VIRICIDAL EFFECT OF LACTOBACILLUS ACIDOPHILUS ON HUMAN IMMUNODEFICIENCY VIRUS TYPE 1 : POSSIBLE ROLE IN HETEROSEXUAL TRANSMISSION.'**
- **CHEMICAL ABSTRACTS, vol. 74, no. 13, 29 March 1971, Columbus, Ohio, US; abstract no. 62368n, S.J. KLEBANOFF ET AL. 'SOURCE OF H2O2 FOR THE UTERINE FLUID-MEDIATED SPERM-INHIBITORY SYSTEM.' page 146 ; & BIOL. REPROD. vol. 3, no. 2 , 1970 pages 236 - 242**
- **CHEMICAL ABSTRACTS, vol. 74, no. 19, 10 May 1971, Columbus, Ohio, US; abstract no. 96628n, D.C. SMITH ET AL. 'UTERINE FLUID-MEDIATED SPERM-INHIBITORY SYSTEM.' page 216 ; & BIOL. REPROD. vol. 3, no. 2 , 1970 pages 229 - 235**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

The present invention relates to compositions for the prevention or treatment of sexually transmitted diseases and use of the compositions for manufacture of a medicament for the prevention or treatment of sexually transmitted diseases. More particularly, the invention relates to compositions using oxidase-haloperoxidase microbicidal activity which is activated by a substrate present in semen. The system is highly efficient and directed in that the binary components required for microbicidal action, i.e., the oxidase-haloperoxidase and a semen substrate for the oxidase, are combined at the time of sexual activity.

Sexually transmitted diseases (STDs) rank among the most common communicable diseases in the world. Despite organized attempts to educate the public, STDs -- such as nonspecific urethritis, chlamydial infections, genital and anorectal herpes and warts, syphilis, gonorrhea, chancroid, and granuloma inguinale -- continue to represent a significant public health problem. For gonorrhea, it is estimated that greater than 250 million persons worldwide, and close to 3 million in the United States, are infected annually. For syphilis, annual worldwide incidence is estimated at 50 million persons, with 400,000 in the United States annually needing treatment. Other infections, including salmonellosis, shigellosis, campylobacter, hepatitis A, B and C, and cytomegalovirus infection, sometimes are sexually transmitted. Strong associations between cervical cancer and herpes viruses and papillomaviruses have been discovered. More recently, the spread of human immunodeficiency virus (HIV) resulting in fatal acquired immunodeficiency syndrome (AIDS) has spread rapidly in homosexual and heterosexual groups.

STD incidence has risen despite advances in the diagnosis and treatment of these diseases. Factors that have contributed to this trend include changes in sexual behavior, e.g., widespread use of contraceptive pills and devices; more varied sexual practices, including orogenital and anorectal contact; emergence of strains of organisms less sensitive to antibiotics; symptomless carriers of infecting agents; a highly mobile population; a high level of sexual activity involving multiple partners; ignorance of the facts by physicians and the public; and reticence of patients in seeking treatment.

Conventional treatment of STDs has generally been limited to the administration of antibiotic, antifungal or antiviral drugs, such as tetracycline, penicillin, metronidazole, nystatin, miconazole, sulphamethoxazole and acyclovir, after the transmission and diagnosis of the STD has been made. Conventional prophylaxis for STDs has generally been limited to the use of physical barriers, such as condoms, that prevent or diminish the exchange of bodily fluids during sexual intercourse. Although certain advances have been made in the diagnosis and treatment of STDs, a strong need exists for new methods and compositions that prevent infection by pathogenic microbes during and immediately following sexual activity. Even in the case of curable STDs, prevention is highly preferable to symptomic diagnosis and treatment.

It has now been discovered that sexually transmitted diseases can be prevented or treated by compositions defined in claim 1 which are effective, in the presence of semen, to inhibit the transmission and colonization of pathogenic microbes present in sexually transmitted fluids. In the presence of semen, the oxidase catalyzes the production of hydrogen peroxide, which in turn is utilized by the haloperoxidase to selectively inhibit pathogenic microbes present in the sexually transmitted fluid without destroying the normal vaginal flora. As such, the present invention provides an antimicrobial system effective against sexually transmitted pathogenic microbes that is activated by sexual activity. The haloperoxidase oxidase system of the invention may optionally be employed at relatively high concentration levels to obtain spermicidal properties.

Suitable haloperoxidases for use in the compositions of the invention include myeloperoxidase (MPO) and eosinophil peroxidase (EPO). Suitable oxidases are choline oxidase, cholesterol oxidase, *l*-amino acid oxidase, polyamine oxidase, urate oxidase (uricase), pyruvate oxidase and lactate oxidase.

The present invention is directed to compositions and use of the compositions for the manufacture of a medicament for treating a human or animal to inhibit sexually transmitted disease by administering to the human or animal amounts of a haloperoxidase and an oxidase which are effective, in the presence of semen, to inhibit the transmission and colonization of pathogenic microbes. In the practice of the invention, the haloperoxidase and the oxidase are provided in the environment of transmitted seminal fluids in a manner in which the antimicrobial properties of the oxidase-haloperoxidase system are triggered by the presence of semen. As a result, maximum microbicidal action is temporally linked to the period of maximum infectious exposure.

Haloperoxidases useful in the present invention are defined as halide:hydrogen peroxide oxidoreductases (e.g., EC No. 1.11.1.7 and EC No. 1.11.1.10 under the International Union of Biochemistry) for which halide is the electron donor or reductant and peroxide is the electron receiver or oxidant. As described in detail in PCT International Publication No. WO 92/14484, mammalian haloperoxidases, such as myeloperoxidase (MPO) and eosinophil peroxidase (EPO), can be used to selectively bind to and, in the presence of peroxide and halide, inhibit the growth of target microbes without eliminating desirable microbes or significantly damaging other components, such as host cells, in the environment of the target microbe. When a target microbe (e.g., pathogenic bacterium, fungus, or virus) has a binding capacity for haloperoxidase greater than that of the desired microbe (e.g., member of the normal flora) or normal host cells, the target microbe selectively binds the haloperoxidase with little or no binding to the normal flora or normal host cells. In the presence of peroxide and halide, the target-bound haloperoxidase catalyzes halide oxidation and facilitates the disproportionation of peroxide to yield singlet molecular oxygen at the surface of the target microbe in accordance with the following reaction:

$$H_2O_2 + HCl \xrightarrow{\quad\textbf{\textit{haloperoxidase}}\quad} H_2O + HOCl \tag{1a}$$

$$H_2O_2 + HOCl \rightarrow H_2O + HCl + {}^1O_2 \tag{2b}$$

The lifetime of singlet molecular oxygen restricts its reactivity and associated damage to within a 0.2 micron radius of the haloperoxidase generator. As such, damage is restricted to the surface of the target microbe. Thus there is selective killing of the target microbe with minimum collateral damage to desired microbes or normal host cells.

Since the antimicrobial activity of the haloperoxidase requires the presence of a peroxide, it has now been discovered that antimicrobial activity can be linked to and triggered by sexual activity when the haloperoxidase is presented together with an oxidase or oxidases which act upon one or more components of semen to produce peroxide. As such, the practice of the invention provides maximum microbicidal action during the time period of maximum exposure to the infecting microbe. Sexual activity is thereby effectively linked to microbicidal action since a compound in the transferred body fluid (i.e., semen) is the rate limiting substrate for an oxidase present in the compositions of the invention. The oxidase-generated peroxide is the rate limiting substrate for haloperoxidase mediated microbicidal action.

Semen substrate-specific oxidases useful in the practice of the invention are those defined in claim 1 which are capable of utilizing a component of semen as a substrate in the production, either directly or indirectly, of hydrogen peroxide in the environment of transmitted seminal fluids and semen. Semen or ejaculum is a complex viscid fluid of the male reproductive tract consisting of spermatozoa suspended in secretions of the accessory reproductive glands including the testes, epididymides, vas deferens, seminal vesicles, prostate, Cowper's and Littre's glands. The ejaculum can be divided into three major portions. The first portion is prostatic secretion free of spermatozoa and is characterized by a high content of acid phosphatase. The second or middle portion contains spermatozoa, the product of testicular and epididymal secretion. The final portion contains the viscous secretions of the seminal vesicles.

A prostatic proteinase acting on a fibrinogen-like protein from the seminal vesicles is responsible for the coagulation of the semen that immediately follows ejaculation. After a period of approximately ten minutes, this coagulum is in turn liquefied by the action of a plasmin-like prostatic enzyme, and the clot fragments are further hydrolyzed to peptides and amino acids by the action of a chymotrypsin-like enzyme.

The total ejaculate volume is $3.4 \pm 1.6$ ml. 13-33% of the ejaculate originates from the prostate, 46-80% from the seminal vesicles, and about 10% from the epididymides. The pH of the total ejaculate is in the 6.9 to 7.4 range, and the chloride concentration ranges from 28 to 57 mEq/L.

The ejaculate contains several nitrogenous organic compounds of interest with respect to the invention. The choline content of the total ejaculate is relatively high at 5.8 mM (i.e., 0.7 mg/ml) when determined two minutes after ejaculation. More importantly, the concentration of choline continues to rise for several hours after ejaculation. The additional choline is generated by the action of acid phosphatase, a component of the prostatic portion, on phosphorylcholine. Glycerylphosphorylcholine is also present in the range of 2.1 to 3.5 mM, but is relatively stable to hydrolysis (Arrata et al., 1978, *Fert Ster* **30**:329-333).

Amino acids are generated by the action of proteases. By 4 to 6 hours after ejaculation the amino acid concentration has increased to approximately 12 mg/ml. Several polyamines, originating in the prostatic secretion, are present in the ejaculate. The spermine concentration ranges from 0.3 to 7 mM. Putrescine and spermidine are also present in lower concentrations. Uric acid, an end product of purine metabolism, is present at a concentration of approximately 0.4 mM.

The ejaculate contains a spectrum of carbohydrates and metabolites that are of interest with respect to the present invention. These include citrate ($\sim$18 mM), fructose ($\sim$13 mM), sialic acid ($\sim$3 mM), fucose ($\sim$3 mM), pyruvate (3 mM), lactate ($\sim$3 mM), and glucose ($\sim$0.3 mM). Galactose and sorbitol are also present. Ascorbic acid is present in the range of 0.1 to 0.4 mM. Cholesterol, a lipid of interest with respect to the present invention, is present in the ejaculate at a concentration of 1.6 mM.

The ejaculate is normally transmitted to the environment of the vagina during sexual intercourse. Like skin, the surface of the vagina is stratified squamous epithelium overlying deeper connective tissue, but unlike normal skin there are no hair follicles, sebaceous glands or sweat glands. The term "vaginal mucosa" is incorrect in that this surface does not secrete mucus. However, droplets of transudate-like mucoid fluid appear at the surface of the vaginal epithelium during sexual excitement. These droplets coalesce to form a smooth lubricating surface that facilitates coitus. Sexual stimulation results in blood stasis within the venous plexus investing the vagina. This vasocongestion is believed to be the source of the lubricating transudate.

The vaginal epithelium is composed of a basal layer of germinal epithelium adjacent to the basement membrane. This basalar layer is $\sim$ 10 microns thick. Above this is the $\sim$ 14 microns thick parabasalar layer. The intermediate layer is approximately ten cells thick, and during ovulation is the thickest layer ($\sim$ 100 microns) of the epithelium. The cells of the transitional layer, also approximately ten cells thick ($\sim$ 80 microns), show the change from oval to squamous configuration. The superficial layer is composed of approximately ten layers of squamous cells. These outer layers of epithelium serve as a protective barrier against the short lived reactants generated by haloperoxidases.

These squamous cells ultimately cornify, degenerate, desquamate and accumulate in the vagina where they mix with Döderlein bacilli, vaginal transudate and cervical mucus to form an acidic fluid resembling curdled milk. This fluid is the normal secretion of the vagina. The quantity of secretion, estimated by weighing the material absorbed by swabbing the entire vaginal wall, is $0.76 \pm 0.04$ g (Stone & Gable, 1959). The daily weight of vaginal secretion collected by tampons from six hysterectomized women with intact ovaries was $1.9 \pm 0.1$ g/24 hours.

The vaginal fluid contains carbohydrate, aliphatic acids, proteins, peptides, amino acids and other compounds released by the disintegration of desquamated epithelial cells. Cholesterol, a constituent of cell membranes, is also present (Preti et al., 1977). Epithelial glycogen is the major source of saccharide and aliphatic acids. Glycogenolysis by either host cells or bacteria produces the glucose that is ultimately metabolized to lactic acid by Döderlein's bacillus. "Döderlein's bacillus" is actually a collection of gram-positive lactic acid bacteria including *Lactobacillus acidophilus, L. casei, L. fermentum, L. cellobiosus,* and *Leuconostoc mesenteroides.* These organisms do not synthesize cytochromes and rely on a flavoenzyme-based redox metabolism. The lactic acid product of lactobacilli metabolism is largely responsible for the acidity of the healthy, mature vagina, i.e., pH 4 to 5. Of additional importance with respect to this invention, these lactobacilli also produce hydrogen peroxide ($H_2O_2$).

In the presence of normal vaginal flora, myeloperoxidase and eosinophil peroxidase are effectively microbicidal. These lactic acid bacteria produce sufficient acid and peroxide to insure dynamic haloperoxidase-based microbicidal action against target bacteria, fungi and viruses. However, conditions, such as vaginitis, which alter the flora and decrease both acidity and peroxide production, can diminish the action of myeloperoxidase and eosinophil peroxidase. Ultimately myeloperoxidaseleosinophil peroxidase will support the growth and re-establishment of normal flora, but during the time interval required to reinstate the normal flora, optimum myeloperoxidase/eosinophil peroxidase microbicidal action is limited by the availability of peroxide.

In the practice of the present invention, a haloperoxidase preferably myeloperoxidase or eosinophil peroxidase, is provided in the environment of transmitted seminal fluids or semen, together with a semen substrate-specific oxidase capable of triggering haloperoxidase antimicrobicidal activity and thereby preventing the transmission of disease caused by microbes present in the transmitted fluids. The semen substrate-specific oxidase can be any of the oxidases defined in claim 1 which are capable of utilizing a component of semen as a substrate in the production, either directly or indirectly, of hydrogen peroxide in the environment of the transmitted seminal fluids or semen. Due to the composition of semen and the nature of the vaginal environment, as described above, the presently most preferred semen substrate-specific oxidases are selected from the group consisting of choline oxidase, cholesterol oxidase, *l*-amino acid oxidase, polyamine oxidase, urate oxidase (uricase), pyruvate oxidase, and lactate oxidase.

In the practice of the invention, the rate of peroxide production in the environment of the transmitted seminal fluids or semen can be controlled by adjusting the activity of the oxidase included in the compositions of the invention. The actual effectiveness of a unit activity of oxidase depends upon the availability of substrate and environmental conditions such as pH. One unit of oxidase activity will generate about 1 µmol of hydrogen peroxide per minute.

In addition to the inhibition or treatment of sexually transmitted diseases, the methods and compositions of the invention can be designed to enhance or diminish the spermicidal properties of the compositions. If sperm viability is a concern, the concentrations of oxidase and haloperoxidase can be decreased to the lowest levels required for effective microbicidal action in order to minimize collateral damage to sperm. If sperm viability is not desired, the oxidase and haloperoxidase concentrations can be increased so that the resulting peroxide generation is sufficient for haloperoxidase-dependent spermicidal action as well as microbe killing. The proximity and intensity of oxygenation activity would be adequate to insure sperm killing even if sperm binding of haloperoxidase is minimal. The short reactive lifetime of singlet molecular oxygen limits the potential for host damage. The thickness of the squamous epithelium protects the viable vaginal tissue from oxygenation damage. The size of myeloperoxidase (about 140,000 daltons) and eosinophil peroxidase (about 74,000 daltons) presents a physical size barrier to the direct vaginal absorption of these haloperoxidases.

Since the antiseptic activity of the haloperoxidase compositions of the invention involves the reaction of peroxide and halide to form hypohalite, and the reaction of peroxide and hypohalite to form singlet molecular oxygen, as described above, the activity of the compositions of the invention is dependent upon the presence, at the site of infection, of a suitable halide. Suitable halides for use in the methods and compositions of the invention may be bromide or chloride. The use, selection, and amount of halide employed in a particular application will depend upon various factors, such as the haloperoxidase used in the antiseptic composition, the desired therapeutic effect, the availability of peroxide and other factors. When the haloperoxidase is myeloperoxidase, the halide may be bromide or chloride. Since chloride is present in essentially all physiological media at levels sufficient to be nonlimiting as the halide cofactor, an external source of chloride is generally not required and thus the presently most preferable halide for use is chloride. When an external source of chloride is desired, the amount of chloride employed will preferably fall in the range of 10 μmol chloride to 150 μmol chloride per ml of solution to approximate physiological conditions. When the haloperoxidase is eosinophil peroxidase, chloride is relatively ineffective as a cofactor, and accordingly, the preferred halide is bromide. When included in liquid compositions for topical use, the compositions of the invention may comprise from 1 nmol bromide to 20 μmol bromide per ml of liquid composition, more preferably from 10 nmol bromide to 10 μmol bromide per ml of liquid composition, and most preferably from 100 nmol bromide to 1 μmol bromide per ml of liquid composition.

The ratio of halide to peroxide is an important consideration in formulating an effective microbicidal environment. Accordingly, in addition to ensuing effective levels of halide and peroxide at the situs of microbial attack, as described above, it is preferable to practice the methods of the invention at halide:peroxide ratios that provide optimal microbicidal activity. High halide:peroxide ratios tend to slow the rate of haloperoxidase activity, but the microbicidal efficiency of the system is relatively well maintained. However, very low ratios compromise haloperoxidase action and diminish microbicidal activity.

The methods and compositions of the invention can be used to treat a broad spectrum of sexually transmitted infections by pathogenic microbes, without destroying the normal flora. As used herein, "pathogenic microbes" is intended to include pathogenic bacteria, fungi, viral particles, yeast, chlamydia, or protozoans which do not normally reside in the host or which are capable of causing host pathology, and which are capable of being specifically and selectively bound to and killed by haloperoxidases, as described in detail herein. Sexually transmitted microbes which can result in pathogenic infection of a host are well known. Thus, the methods and compositions of the invention can be used in the treatment or prophylaxis of sexually transmitted diseases including, for example, gonococcal urethritis, nonspecific urethritis, mucopurulent cervicitis, nonspecific genital infections, chlamydial infections, syphilis, genital candidiasis, chancroid, lymphogranuloma venereum, genital herpes, acquired immunodeficiency syndrome (AIDS), and the like.

The compositions of the invention generally comprise amounts of a haloperoxidase and a semen substrate-specific oxidase which are effective, in the presence of semen, to inhibit the transmission and colonization of pathogenic microbes, together with a pharmaceutically acceptable carrier or vehicle. The semen substrate-specific oxidase and the haloperoxidase can be free, i.e., separate components of the composition. Alternatively, the oxidase and the haloperoxidase can be covalently or non-covalently linked together or otherwise complexed to ensure close physical proximity between peroxide produced by the oxidase and the haloperoxidase. Any pharmaceutically acceptable carrier or vehicle may be generally used to incorporate the oxidase-haloperoxidase system, provided that the carrier does not significantly interfere with the selective binding capabilities of the haloperoxide or with haloperoxidase or oxidase activities. Preferably, the pharmaceutically acceptable carrier or vehicle is in the form of a liquid, jelly, suppository, foam, sponge, troche or the like containing haloperoxidase and oxidase plus sufficient halide, and adjusted to a pH sufficient to insure optimum microbicidal action. The oxidase-haloperoxidase system can be incorporated into: (a) ointments and jellies, (b) inserts (suppositories, sponges, troches, and the like), (c) foams, and (d) douches. The material is preferably introduced into the vagina of a female, or other environment of transmission of semen or seminal fluids, at about the time of, and preferably prior to sexual intercourse. Depending on the strength of the formulation, the system can be employed for the treatment of nonspecific vaginitis, for protection against sexually transmitted diseases, and for prevention of conception. The manner of administration will preferably be designed to obtain direct contact of the antiseptic compositions with sexually transmitted microbes.

For topical applications, the pharmaceutically acceptable carrier may take the form of lubricants, foams, liquids, creams, lotions, or gels, and may additionally comprise organic solvents, emulsifiers, gelling agents, moisturizers, stabilizers, surfactants, wetting agents, preservatives, time release agents, and minor amounts of humectants, sequestering agents, dyes, perfumes, and other components commonly employed in pharmaceutical compositions for topical administration. Compositions of the invention may be impregnated into absorptive materials, such as sponges, or coated onto the surface of solid phase materials, such as condoms, to deliver the compositions to the environment of ejaculated seminal fluids or semen during or after sexual intercourse. Other delivery systems of this type will be readily apparent to those skilled in the art.

Solid dosage forms for topical administration include suppositories, powders, and granules. In solid dosage forms, the compositions may be admixed with at least one inert diluent such as sucrose, lactose, or starch, and may additionally comprise lubricating agents, buffering agents and other components well known to those skilled in the art.

Actual dosage levels of haloperoxidase and the semen-substrate specific oxidase in the compositions of the invention may be varied so as to obtain amounts of haloperoxidase and oxidase at the site of sexually transmitted fluids to obtain the desired therapeutic or prophylactic response for a particular haloperoxidase and method of administration. Accordingly, the selected dosage level will depend on the nature and site of infection, the desired therapeutic response, the route of administration, the desired duration of treatment and other factors. Generally, when the haloperoxidase is myeloperoxidase, liquid dosage forms for topical, vaginal, anal or buccal administration will comprise from 0.1 picomoles (pmol) to 500 pmol of myeloperoxidase per ml of liquid composition, more preferably from 1 pmol to 200 pmol of myeloperoxidase per ml of liquid composition, and most preferably from 1 pmol to 50 pmol of myeloperoxidase per ml of liquid composition. Similar dosages of eosinophil peroxidase may be employed. For non-liquid compositions, dosages of either haloperoxidase will generally comprise from 1 pmol to 1 nmol of haloperoxidase (myeloperoxidase or eosinophil peroxidase) per gram of composition, more preferably from 10 pmol to 500 pmol of haloperoxidase per gram of composition, and most preferably from 50 pmol to 250 pmol of haloperoxidase per gram of composition. Within the foregoing general parameters, from 0.1 pmol to 20 pmol haloperoxidase (myeloperoxidase or eosinophil peroxidase) per milliliter are preferred for microbicidal action, while from about 20 pmol to about 500 pmol haloperoxidase are preferred for both microbicidal and spermicidal action. While the foregoing dosage ranges represent general guidelines and presently preferred embodiments, the actual microbicidal and spermicidal activity of the system will be highly dependent on oxidase generation of peroxide, the environment of application and other factors.

Oxidase dosage is described in terms of activity and expressed as units. One unit is defined as the quantity of oxidase that generates 1 $\mu$mol of hydrogen peroxide per minute. If the availability of substrate in semen is not limiting, the rate of peroxide generation can be adjusted by controlling the activity of the oxidase in the formulation. Oxidase activity is dependent on the type and quantity of substrate available and on environmental factors such as temperature and pH and including components present in the formulation. Generally, liquid (or non-liquid) dosage forms will comprise a sufficient amount of semen substrate-specific oxidase to generate from 1 nmol to 2 $\mu$mol peroxide per ml (or per gram) per minute when in the presence of semen, and more preferably from 10 nmol to 500 nmol peroxide per ml (or gram) per minute. The foregoing amounts correspond to from 1 milliunit to 2 units of oxidase activity, and from 10 milliunits to 500 muliunits of oxidase activity, respectively. The lower rates of peroxide generation, e.g., from 1 nmol to 100 nmol peroxide per minute will generally be effective for microbicidal action, while the higher rates of peroxide generation, e.g., from 100 nmol to 2 $\mu$mol peroxide per minute are preferred when both microbicidal and spermicidal activity are desired.

As used herein, the term "normal flora" means bacteria which normally reside in or on body surfaces of a healthy host at symbiotic levels. Normal flora include, for example, the lactic acid family of bacteria in the mouth or vagina of human subjects, e.g., *Streptococcus* (viridans) in the mouth, and *Lactobacillus sp.* (e.g., Tissier's bacillus and Döderlein's bacillus) in the intestines of breast-fed infants, external genitalia, anterior urethra and vagina. Microorganisms which constitute normal flora of a host are well known (e.g., see *Principles and Practice of Infectious Diseases,* supra, New York, pp. 34-36 and 161). It has been found that the haloperoxidases of the invention selectively bind to many pathogenic microbes in preference over normal flora. The human or animal is preferably treated in accordance with the invention with amounts of haloperoxidase and semen substrate-specific oxidase which are ineffective to eliminate normal flora from the human or animal.

## EXAMPLES

### Example 1

### *Use of Choline Oxidase with Haloperoxidase.*

As described above, semen contains approximately 6 mM (6 $\mu$mol/ml) choline. Since the average ejaculate volume is 3.5 ml, approximately 21 $\mu$mol of choline are delivered in the ejaculate.

Choline is a substrate for choline oxidase (*choline:O$_2$ oxidoreductase* EC 1.1.3.17):

$$\text{choline} + O_2 \longrightarrow \boldsymbol{\textit{choline oxidase}} \rightarrow \text{betaine aldehyde} + H_2O_2 \tag{2a}$$

$$\text{betaine aldehyde} + O_2 \longrightarrow \boldsymbol{\textit{choline oxidase}} \rightarrow \text{betaine} + H_2O_2 \tag{2b}$$

Note that two peroxides are formed per choline oxidized to betaine. Bacterial choline oxidase is commercially available. The choline oxidase from *Alcaligenes is a* flavine adenine dinucleotide enzyme of approximately 70,000 daltons molecular weight. Its Michaelis constant (Km) is 0.9 mM for choline and 6.2 mM for betaine aldehyde (Ohta-Fukuyama et al., 1980, *J Biochem* **88**:197-203). The choline oxidase from *Arthrobacter* is a flavoenzyme of approximately 75,000 daltons with an isoelectric point (pI) of 4.5. Its Km is 1.2 mM for choline and 8.7 mM for betaine aldehyde (Ikuta et al., 1977, *J Biochem* **82**:1741-7149).

In addition to choline, the semen contains approximately 14 mM phosphorylcholine that is converted to choline by the action of acid phosphatase:

$$\text{phosphorylcholine} \xrightarrow{\textit{acid phosphatase}} \text{choline} + \text{phosphate} \tag{3}$$

The hydrolysis of semen phosphorylcholine is essentially complete in one hour. As such, the availability of choline continues to increase within the first hour after ejaculation (Arrata et al., 1978, *Fertil Steril* **30**:329-333). Within the first hour after ejaculation the total choline, i.e., the initial choline plus hydrolyzed phosphorylcholine, available is approximately 20 µmol/ml of semen or approximately 70 µmol total.

Total choline conversion by choline oxidase would yield 140 µmol hydrogen peroxide. This is a relatively large quantity of peroxide, but choline conversion to peroxide is dependent on the activity of choline oxidase in the formulation, and as such, the rate of peroxide generation can be adjusted by controlling the quantity of choline oxidase in the formulation. Choline oxidase can be adjusted to a concentration yielding a rate of peroxide generation sufficient for microbicidal action but insufficient for spermicidal action. If desired, the choline oxidase concentration could be increased to yield a rate of peroxide generation sufficient for both microbicidal and spermicidal action. Since choline is not a major constituent of the vaginal fluid, choline oxidation dependent oxygenation activity will terminate with exhaustion of the semen choline.

The bacterial, yeast and fungal spore microbicidal capacities of the choline oxidase-myeloperoxidase and choline oxidase-eosinophil peroxidase systems were demonstrated as follows: Reaction mixtures were prepared containing 0.2 units (i.e., 20 µg) choline oxidase from *Alcaligenes sp.* (when present, as indicated), 20 pmol (2.8 µg) porcine myeloperoxidase (Lot#1899201, ExOxEmis Inc., San Antonio, Texas) or 20 pmol (1.5 µg) porcine eosinophil peroxidase (Lot#1929201, ExOxEmis Inc., San Antonio, Texas) in 50 mM Acetate Buffer containing 100 mEq/L Cl⁻, 1 mEq/L Br⁻, and 1 mM *l*-alanine. The pH was adjusted to 7 by addition of 50 mM MOPS buffer. The final concentration of choline was 150 mM (150 µmol/ml). The final volume was 1 ml. The mixtures were inoculated with $\sim 10^6$ to $10^7$ colony forming units (CFU) of *S. aureus, C. albicans* or *A. fumigatus* spores, and were incubated for four hours at 22°C. *S. aureus* was then plated on trypticase soy agar. *C. albicans* and *A. fumigatus* were plated on Sabouraud's dextrose agar. The results are expressed in Table 1 as the CFU's counted after $\sim$ 48 hours of incubation at 35°C.

Table 1

| Choline Oxidase-Haloperoxidase Microbicidal Action Against *Staphylococcus aureus, Candida albicans, and Aspergillus fumigatus*: | | | |
|---|---|---|---|
| **Organism** | **Choline Oxidase** | **Haloperoxidase** | **CFU** |
| *Staph. aureus* | None | None | 19,400,000 |
| *Staph. aureus* | 0.2 Unit | None | 15,400,000 |
| *Staph. aureus* | 0.2 Unit | 20 *p*mol MPO | 0 |
| *Staph. aureus* | 0.2 Unit | 20 *p*mol EPO | 0 |
| *Cand. albicans* | None | None | 1,460,000 |
| *Cand. albicans* | 0.2 Unit | None | 1,200,000 |
| *Cand. albicans* | 0.2 Unit | 20 *p*mol MPO | 0 |
| *Cand. albicans* | 0.2 Unit | 20 *p*mol EPO | 0 |
| *Asperg. fumigatus* | None | None | 1,260,000 |
| *Asperg. fumigatus* | 0.2 Unit | None | 1,300,000 |
| *Asperg. fumigatus* | 0.2 Unit | 20 *p*mol MPO | 0 |
| *Asperg. fumigatus* | 0.2 Unit | 20 *p*mol EPO | 0 |

As shown in Table 1, when substrate (i.e., choline) is not limiting (e.g., 150 mM), 0.2 units (20 µg) of choline oxidase plus either 20 pmol (2.8 µg) myeloperoxidase or 20 pmol (1.5 µg) eosinophil peroxidase is sufficient for complete killing of *Staphylococcus aureus, Candida albicans,* as well as *Aspergillus fumagatus* spores. The quantity of *choline oxidase-haloperoxidase* required for effective spermicidal action must be empirically determined for each type of formulation employing this system.

The direct effect of varying choline oxidase on microbicidal activity against *Staphylococcus aureus* was determined as follows. In order to assess the choline oxidase requirement, the foregoing procedure was followed except that the quantities of choline (40 μmol/ml) and haloperoxidase (10 *p*mol/ml) were set to lower but still within non-rate-limiting concentrations, and choline oxidase was varied.

Each test contained the indicated quantity of choline oxidase from *Alcaligenes sp.* (0.1 Unit equals 10 μg), 10 pmol (1.4 μg) porcine myeloperoxidase (Lot#1899201) or 10 pmol (0.7 μg) porcine eosinophil peroxidase (Lot#1929201) in 50 mM Acetate Buffer containing 100 mEq/L Cl⁻, 1 mEq/L Br⁻, and 1 mM *l*-alanine. The pH was adjusted to 6.7 by addition of 50 mM MOPS buffer. The final concentration of choline was 40 mM (40 μmol/ml). The final volume was 1 ml. After two hours incubation (37°C) the microbes were plated on trypticase soy agar. The results are expressed in Table 2 as the colony forming units (CFU's) counted.

Table 2

| Effect of Choline Oxidase Concentration on Choline Oxidase- Haloperoxidase Microbicidal Action Against *Staphylococcus aureus:* | | | |
|---|---|---|---|
| **Organism** | **Choline Oxidase** | **Haloperoxidase** | **CFU** |
| *Staph. aureus* | None | None | 23,800,000 |
| *Staph. aureus* | 0.2 Unit | None | 8,200,000 |
| *Staph. aureus* | 0.2 Unit | 10 *p*mol MPO | 0 |
| *Staph. aureus* | 0.1 Unit | 10 *p*mol MPO | 0 |
| *Staph. aureus* | 0.05 Unit | 10 *p*mol MPO | 0 |
| *Staph. aureus* | 0.025 Unit | 10 *p*mol MPO | 80,000 |
| *Staph. aureus* | None | 10 *p*mol MPO | 17,000,000 |
| *Staph. aureus* | 0.2 Unit | 10 *p*mol EPO | 0 |
| *Staph. aureus* | 0.1 Unit | 10 *p*mol EPO | 0 |
| *Staph. aureus* | 0.05 Unit | 10 *p*mol EPO | 0 |
| *Staph. aureus* | 0.025 Unit | 10 *p*mol EPO | 32,000 |
| *Staph. aureus* | None | 10 *p*mol EPO | 2,400,000 |

As shown in Table 2, complete killing *of Staph. aureus* is observed with 0.05 Units of oxidase, and incomplete but significant killing is observed with 0.025 units of oxidase. Assuming that optimum reaction requirements are met, 50 milliunits (0.05 unit) of oxidase should generate about 50 nmol hydrogen peroxide per minute. Some killing of *Staph. aureus* is also observed with eosinophil peroxidase alone, and to a lesser extent, with choline oxidase alone.

**Example 2**

***Use of Cholesterol Oxidase with Haloperoxidase.***

The cholesterol concentration of semen is approximately 1.6 mM. Assuming a 3.5 ml volume, there are approximately 5.6 μmol cholesterol per ejaculum. Although difficult to quantify, cholesterol is also a major component of the vaginal fluid (Stone & Gable, 1959).

Cholesterol and other 3β-hydroxy steroids are the substrates for the flavoenzyme cholesterol oxidase (*cholesterol:$O_2$ oxidoreductase* EC 1.1.3.6):

$$\text{Cholesterol} + O_2 \underline{\quad\textit{\textbf{cholesterol axidase}}\quad}\rightarrow \text{4-cholesten-3-one} + H_2O_2 \qquad (4)$$

A broad variety of microbial cholesterol oxidases are commercially available. For the present example, *Norcardia erythropolis* cholesterol oxidase was employed. This flavoenzyme is very stable, active over a broad pH range, and has a Km of 14 mM for cholesterol (Richmond, 1973, *Clin Chem* **19**:1350-1356).

The *cholesterol oxidase-haloperoxidase system* differs from the previously described *choline oxidase-haloperoxidase system* in that cholesterol, the substrate for the oxidase, is present in the vaginal fluid prior to exposure to semen. *Cholesterol oxidase-haloperoxidase* activity is in part independent of semen, and as such, microbicidal action is initiated at the time of application. In the event of sexual intercourse, ejaculation of semen results in the coincident introduction of ∼ 5.6 μmol of cholesterol as substrate for the *cholesterol oxidase-haloperoxidase* formulation in place in the vagina.

Cholesterol oxidase is a relatively robust enzyme, and as demonstrated by the data of Table 3, the *cholesterol oxidase-haloperoxidase system* exerts a potent microbicidal action against bacteria, yeast and fungal spores. Even if the combined vaginal plus semen cholesterol does not exceed 6 μmol total, this system should provide adequate protection against sexually transmitted diseases. This system is also potentially spermicidal, but the limitation of substrate suggests that it might be less potent than the *choline oxidase-haloperoxidase system* in this regard.

To demonstrate the efficacy of compositions of the invention employing cholesterol oxidase as the semen-specific oxidase, the procedure of Example 1 was repeated using cholesterol oxidase as follows. Where indicated the reaction contained 0.1 units (i.e., 4 μg) cholesterol oxidase from *Norcardia erythropolis,* 20 pmol (2.8 μ g) porcine myeloperoxidase (Lot#1899201, ExOxEmis, Inc., San Antonio, Texas) or 20 pmol (1.5 μg) porcine eosinophil peroxidase (Lot#1929201, ExOxEmis, Inc., San Antonio, Texas) in 50 mM Acetate Buffer containing 100 mEq/L Cl⁻, 1 mEq/L Br⁻, and 1 mM *l*-alanine. The pH was adjusted to 7 by addition of 50 mM MOPS buffer. The final concentration of cholesterol was 7 mM (7 μmol/ml). The final volume was 1 ml. After four hours incubation the microbes were plated. *S. aureus* was plated on trypticase soy agar. *C. albicans* and *A. fumigatus* were plated on Sabouraud's dextrose agar. The results are expressed in Table 3 as the colony forming units (CFU's) counted.

Table 3

| Cholesterol Oxidase-Haloperoxidase Microbicidal Action Against *Staphylococcus aureus, Candida albicans, and Aspergillus fumigatus*: | | | |
|---|---|---|---|
| **Organism** | **Cholesterol Oxidase** | **Haloperoxidase** | **CFU** |
| *Staph. aureus* | None | None | 19,400,000 |
| *Staph. aureus* | 0.1 Unit | None | 29,200,000 |
| *Staph. aureus* | 0.1 Unit | 20 *p*mol MPO | 0 |
| *Staph. aureus* | 0.1 Unit | 20 *p*mol EPO | 0 |
| *Cand. albicans* | None | None | 1,460,000 |
| *Cand. albicans* | 0.1 Unit | None | 1,580,000 |
| *Cand. albicans* | 0.1 Unit | 20 *p*mol MPO | 0 |
| *Cand. albicans* | 0.1 Unit | 20 *p*mol EPO | 0 |
| *Asperg. fumigatus* | None | None | 1,260,000 |
| *Asperg. fumigatus* | 0.1 Unit | None | 880,000 |
| *Asperg. fumigatus* | 0.1 Unit | 20 *p*mol MPO | 0 |
| *Asperg. fumigatus* | 0.1 Unit | 20 *p*mol EPO | 0 |

**Example 3**

***Use of Alternative Oxidases with Haloperoxidase.***

The semen contains a number of other compounds that are substrates, or can be modified to serve as substrates, for oxidases yielding peroxide. The more obvious compounds include *l*-amino acids, polyamines, urate, lactate, pyruvate, glucose and galactose. The concentrations of these compounds in semen are sufficient to drive oxidase-haloperoxidase microbicidal action. Certain of these compounds, such as *l*-amino acids and polyamines, have advantage with regard to being present in relatively high concentrations.

A number of potential substrates are also present if an additional enzyme is incorporated in the formulation. For example, if an isomerase were found to convert fructose to glucose, inclusion of such an enzyme in a *glucose oxidase-haloperoxidase* formulation would allow fructose (~13 μmol/ml semen) to serve as an indirect substrate for the generation of peroxide by glucose oxidase. In addition, depriving the spermatozoa of fructose, a major nutrient, is likely to further contribute to the spermicidal capacity of such a system.

Glucose is a major nutrient for Döderlein's bacilli, and as such, glucose is probably not the best substrate for an oxidase-haloperoxidase microbicidal system. Enzymatic depletion of glucose might adversely affect the normal flora. It is far better to have Döderlein's bacilli directly metabolize glucose to lactic acid and peroxide. However, the lactate product of normal flora metabolism can serve as substrate for a *lactate oxidase-haloperoxidase* microbicidal system.

Lactate is substrate for the flavoenzyme lactate oxidase (*lactate:$O_2$ oxidoreductase*):

$$\text{lactate} + O_2 \xrightarrow{\quad\text{lactate oxidase}\quad} \text{pyruvate} + H_2O_2 \qquad\qquad (5)$$

A commercial lactate oxidase prepared from *Pediococcus sp.* was used in the present example (Mizutani et al., 1983, *Anal Chem* **55**:35-38). As illustrated by the data of Table 4, this lactate oxidase-haloperoxidase system demonstrated good bactericidal activity. However, fungicidal activity is poor in comparison to the other *oxidase-haloperoxidase systems* tested.

The procedure of Example 1 was repeated using lactate oxidase as the semen-substrate specific oxidase as follows. Where indicated the reaction contained 0.2 units (i.e., 5 µg) lactate oxidase from *Pediococcus sp.*, 20 pmol (2.8 µg) porcine myeloperoxidase (Lot#1899201, ExOxEmis, Inc., San Antonio, Texas) or 20 pmol (1.5 µg) porcine eosinophil peroxidase (Lot#1929201, ExOxEmis, Inc., San Antonio, Texas) in 50 mM Acetate Buffer containing 100 mEq/L Cl⁻, 1 mEq/L Br⁻, and 1 mM *l*-alanine. The pH was adjusted to 7 by addition of 50 mM MOPS buffer. The final concentration of lactate was 150 mM (150 µmol/ml). The final volume was 1 ml. After four hours incubation the microbes were plated. *S. aureus* was plated on trypticase soy agar. *C. albicans* and *A. fumigatus* were plated on Sabouraud's dextrose agar. The results are expressed in Table 4 as the colony forming units (CFU's) counted.

Table 4

| Lactate Oxidase-Haloperoxidase Microbicidal Action Against *Staphylococcus aureus, Candida albicans, and Aspergillus fumigatus*: | | | |
|---|---|---|---|
| Organism | Lactate Oxidase | Haloperoxidase | CFU |
| *Staph. aureus* | None | None | 19,400,000 |
| *Staph. aureus* | 0.2 Unit | None | 24,400,000 |
| *Staph. aureus* | 0.2 Unit | 20 *p*mol MPO | 0 |
| *Staph. aureus* | 0.2 Unit | 20 *p*mol EPO | 0 |
| *Cand. albicans* | None | None | 1,460,000 |
| *Cand. albicans* | 0.2 Unit | None | 1,020,000 |
| *Cand. albicans* | 0.2 Unit | 20 *p*mol MPO | 1,500,000 |
| *Cand. albicans* | 0.2 Unit | 20 *p*mol EPO | 1,180,000 |
| *Asperg. fumigatus* | None | None | 1,260,000 |
| *Asperg. fumigatus* | 0.2 Unit | None | 760,000 |
| *Asperg. fumigatus* | 0.2 Unit | 20 *p*mol MPO | 400,000 |
| *Asperg. fumigatus* | 0.2 Unit | 20 *p*mol EPO | 18,000 |

## Claims

1. A pharmaceutical composition for inhibiting the growth, in the vagina of a female host, of pathogenic bacteria, *Chlamydia* or yeast that cause sexually transmitted disease, comprising amounts of a haloperoxidase and an oxidase selected from the group consisting of choline oxidase, cholesterol oxidase, *l*-amino acid oxidase, polyamine oxidase, urate oxidase, pyruvate oxidase, and lactate oxidase which are effective in the presence of seminal fluids or semen, to inhibit the growth of the pathogenic bacteria, *Chlamydia* or yeast, together with a pharmaceutically acceptable carrier.

2. The composition of Claim 1 wherein the haloperoxidase is selected from the group consisting of myeloperoxidase and eosinophil peroxidase.

3. The composition of Claim 2 which comprises from 0.1 pmol of myeloperoxidase or eosinophil peroxidase to 500 pmol of myeloperoxidase or eosinophil peroxidase per ml in a liquid pharmaceutically acceptable carrier.

4. The composition of Claim 2 which comprises from 1 pmol of myeloperoxidase or eosinophil peroxidase to 1 nmol of myeloperoxidase or eosinophil peroxidase per gram in a non-liquid pharmaceutically acceptable carrier.

5. The composition of Claim 2 which comprises from 1 pmol of myeloperoxidase or eosinophil peroxidase to 200 pmol of myeloperoxidase or eosinophil peroxidase per ml in a liquid pharmaceutically acceptable carrier.

6. The composition of Claim 2 which comprises from 10 pmol of myeloperoxidase or eosinophil peroxidase to 500 pmol of myeloperoxidase or eosinophil peroxidase per gram in a non-liquid pharmaceutically acceptable carrier.

7. The composition of Claim 1 which comprises from 1 milliunits to 2 units of semen substrate-specific oxidase per ml in a liquid pharmaceutically acceptable carrier.

8. The composition of Claim 1 which comprises from 1 milliunit of semen substrate-specific oxidase to 2 units semen substrate-specific oxidase per gram in a non-liquid pharmaceutically acceptable carrier.

9. The composition of Claim 1 wherein the semen substrate-specific oxidase is selected from the group consisting of choline oxidase, cholesterol oxidase, and lactate oxidase.

10. The composition of Claim 9 which comprises from 1 milliunits to 2 units of choline oxidase, cholesterol oxidase per ml in a liquid pharmaceutically acceptable carrier.

11. The composition of Claim 9 which comprises from 1 milliunits to 2 units of choline oxidase, cholesterol oxidase per gram in a non-liquid pharmaceutically acceptable carrier.

12. The composition of Claim 9 which comprises 10 milliunits to 500 milliunits of choline oxidase, cholesterol oxidase per ml in a liquid pharmaceutically acceptable carrier.

13. The composition of Claim 9 which comprises 10 milliunits to 500 milliunits of choline oxidase, cholesterol oxidase per gram in a non-liquid pharmaceutically acceptable carrier.

14. The composition of Claim 1 wherein the pharmaceutically acceptable carrier is in the form of an ointment, jelly, foam, douche or vaginal insert.

15. The composition of Claim 1 wherein the haloperoxidase is covalently linked to the oxidase.

16. The composition of Claim 1 wherein the haloperoxidase is non-covalently linked to the oxidase.

17. Use of a composition of a haloperoxidase and an oxidase selected from the group consisting of choline oxidase, cholesterol oxidase, *l*-amino acid oxidase, polyamine oxidase, urate oxidase, pyruvate oxidase, and lactate oxidase in a pharmaceutically acceptable carrier for the manufacture of a medicament effective in the presence of seminal fluids or semen to inhibit the growth of pathogenic bacteria, *Chlamydia* or yeast.

18. The use of Claim 17 wherein the haloperoxidase is selected from the group consisting of myeloperoxidase and eosinophil peroxidase.

19. The use of Claim 18 wherein the pathogenic bacteria, *Chlamydia* or yeast are contacted with from 0.1 pmol of myeloperoxidase or eosinophil peroxidase to 500 pmol of myeloperoxidase or eosinophil peroxidase per ml in a liquid pharmaceutically acceptable carrier.

20. The use of Claim 18 wherein the pathogenic bacteria, *Chlamydia* or yeast are contacted with from 1 pmol of myeloperoxidase or eosinophil peroxidase to 200 pmol of myeloperoxidase or eosinophil peroxidase per ml in a liquid pharmaceutically acceptable carrier.

21. The use of Claim 18 wherein the pathogenic bacteria, *Chlamydia* or yeast are contacted with from 1 pmol of myeloperoxidase or eosinophil peroxidase to 1 nmol of myeloperoxidase or eosinophil peroxidase per gram in a non-liquid pharmaceutically acceptable carrier.

22. The use of Claim 18 wherein the pathogenic bacteria, *Chlamydia* or yeast are contacted with from 10 pmol of myeloperoxidase or eosinophil peroxidase to 500 pmol of myeloperoxidase or eosinophil peroxidase per gram in a non-liquid pharmaceutically acceptable carrier.

23. The use of Claim 17 wherein the oxidase is effective to generate from 1 nmol to 1 µmol peroxide per ml per minute when in the presence of semen.

**24.** The use of Claim 17 wherein the oxidase is effective to generate from 10 nmol to 500 nmol peroxide per ml per minute when in the presence of semen.

**25.** The use of Claim 17 wherein the oxidase is selected from the group consisting of choline oxidase, cholesterol oxidase, and lactate oxidase.

**26.** The use of Claim 25 wherein the pathogenic bacteria, *Chlamydia* or yeast are contacted with from 1 milliunit to 2 units of choline oxidase or cholesterol oxidase per ml in a liquid pharmaceutically acceptable carrier.

**27.** The use of Claim 25 wherein the pathogenic bacteria, *Chlamydia* or yeast are contacted with from 1 milliunit to 2 units of choline oxidase or cholesterol oxidase per gram in a non-liquid pharmaceutically acceptable carrier.

**28.** The use of Claim 25 wherein the pathogenic bacteria, *Chlamydia* or yeast are contacted with 10 milliunits to 500 milliunits of choline oxidase or cholesterol oxidase per ml in a liquid pharmaceutically acceptable carrier.

**29.** The use of Claim 25 wherein the pathogenic bacteria, *Chlamydia* or yeast are contacted with from 10 milliunits to 500 milliunits of choline oxidase or cholesterol oxidase per gram in a non-liquid pharmaceutically acceptable carrier.

**30.** The use of Claim 17 wherein the pathogenic bacteria, *Chlamydia* or yeast are contacted with the haloperoxidase and the oxidase in an acceptable ointment, jelly, foam, douche or vaginal insert.

**Patentansprüche**

**1.** Pharmazeutische Zusammensetzung zur Hemmung des Wachstums von krankheitserregenden Bakterien, *Chlamydia* oder Hefe in der Scheide eines weiblichen Wirtes, die sexuell übertragbare Krankheiten verursachen, die Mengen an Haloperoxidase und einer aus der aus Cholinoxidase, Cholesterinoxidase, *l*-Aminosäureoxidase, Polyaminoxidase, Uratoxidase, Pyruvatoxidase und Laktatoxidase bestehenden Gruppe ausgewählten Oxidase enthält, die bei Vorhandensein von Samenflüssigkeit oder Sperma so wirken, daß sie das Wachstum von krankheitserregenden Bakterien, *Chlamydia* oder Hefe gemeinsam mit einem pharmazeutisch annehmbaren Träger hemmen.

**2.** Zusammensetzung nach Anspruch 1,
**dadurch gekennzeichnet, daß**
die Haloperoxidase aus der aus Myeloperoxidase und eosinophiler Peroxidase bestehenden Gruppe ausgewählt wird.

**3.** Zusammensetzung nach Anspruch 2,
**dadurch gekennzeichnet, daß**
sie von 0,1 pmol Myeloperoxidase oder eosinophiler Peroxidase bis zu 500 pmol Myeloperoxidase oder eosinophile Peroxidase pro ml in einem flüssigen, pharmazeutisch annehmbaren Träger enthält.

**4.** Zusammensetzung nach Anspruch 2,
**dadurch gekennzeichnet, daß**
sie von 1 pmol Myeloperoxidase oder eosinophiler Peroxidase bis zu 1 nmol Myeloperoxidase oder eosinophile Peroxidase pro Gramm in einem nichtflüssigen, pharmazeutisch annehmbaren Träger enthält.

**5.** Zusammensetzung nach Anspruch 2,
**dadurch gekennzeichnet, daß**
sie von 1 pmol Myeloperoxidase oder eosinophiler Peroxidase bis zu 200 pmol Myeloperoxidase oder eosinophile Peroxidase pro ml in einem flüssigen, pharmazeutisch annehmbaren Träger enthält.

**6.** Zusammensetzung nach Anspruch 2,
**dadurch gekennzeichnet, daß**
sie von 10 pmol Myeloperoxidase oder eosinophiler Per-oxidase bis zu 500 pmol Myeloperoxidase oder eosinophile Peroxidase pro Gramm in einem nichtflüssigen, pharmazeutisch annehmbaren Träger enthält.

**7.** Zusammensetzung nach Anspruch 1,
**dadurch gekennzeichnet, daß**
sie 1 Millieinheit bis 2 Einheiten spermasubstratspezifische Oxidase pro ml in einem flüssigen, pharmazeutisch annehmbaren Träger enthält.

**8.** Zusammensetzung nach Anspruch 1,
**dadurch gekennzeichnet, daß**
sie 1 Millieinhe spermasubstratspezifische Oxidase bis zu 2 Einheiten spermasubstratspezifische Oxidase pro Gramm in einem nichtflüssigen, pharmazeutisch annehmbaren Träger enthält.

**9.** Zusammensetzung nach Anspruch 1,
**dadurch gekennzeichnet, daß**
die spermasubstratspezifische Oxidase aus der aus Cholinoxidase, Cholesterinoxidase und Laktatoxidase bestehenden Gruppe ausgewählt wird.

**10.** Zusammensetzung nach Anspruch 9,
**dadurch gekennzeichnet, daß**
sie 1 Millieinheit bis 2 Einheiten Cholinoxidase, Cholesterinoxidase pro ml in einem flüssigen, pharmazeutisch annehmbaren Träger enthält.

**11.** Zusammensetzung nach Anspruch 9,
**dadurch gekennzeichnet, daß**
sie 1 Millieinhe bis 2 Einheiten Cholinoxidase, Cholesterinoxidase pro Gramm in einem nichtflüssigen, pharmazeutisch annehmbaren Träger enthält.

**12.** Zusammensetzung nach Anspruch 9,
**dadurch gekennzeichnet, daß**
sie 10 Millieinheiten bis 500 Millieinheiten Cholinoxidase, Cholesterinoxidase pro ml in einem flüssigen, pharmazeutisch annehmbaren Träger enthält.

**13.** Zusammensetzung nach Anspruch 9,
**dadurch gekennzeichnet, daß**
sie 10 Millieinheiten bis 500 Millieinheiten Cholinoxidase, Cholesterinoxidase pro Gramm in einem nichtflüssigen, pharmazeutisch annehmbaren Träger enthält.

**14.** Zusammensetzung nach Anspruch 1,
**dadurch gekennzeichnet, daß**
der pharmazeutisch annehmbare Träger in Form einer Salbe, Gallerte, Schaum, Spülung oder eines Elementes zum Einführen in die Scheide vorhanden ist.

**15.** Zusammensetzung nach Anspruch 1,
**dadurch gekennzeichnet, daß**
die Haloperoxidase kovalent mit der Oxidase verbunden ist.

**16.** Zusammensetzung nach Anspruch 1,
**dadurch gekennzeichnet, daß**
die Haloperoxidase nicht-kovalent mit der Oxidase verbunden ist.

**17.** Verwendung einer Zusammensetzung aus einer Haloperoxidase und einer aus der aus Cholinoxidase, Cholesterinoxidase, *l*-Aminosäureoxidase, Polyaminoxidase, Uratoxidase, Pyruvatoxidase und Laktatoxidase bestehenden Gruppe ausgewählten Oxidase in einem pharmazeutisch annehmbaren Träger zur Herstellung eines Medikamentes, das bei Vorhandensein von Samenflüssigkeit oder Sperma so wirkt, daß es das Wachstum von krankheitserregenden Bakterien, *Chlamydia* oder Hefe hemmt.

**18.** Verwendung nach Anspruch 17,
**dadurch gekennzeichnet, daß**
die Haloperoxidase aus der aus Myeloperoxidase und eosinophiler Peroxidase bestehenden Gruppe ausgewählt wird.

**19.** Verwendung nach Anspruch 18,
**dadurch gekennzeichnet, daß**
die krankheitserregenden Bakterien, *Chlamydia* oder Hefe mit 0,1 pmol Myeloperoxidase oder eosinophiler Peroxidase bis zu 500 pmol Myeloperoxidase oder eosinophiler Peroxidase pro ml in einem flüssigen, pharmazeutisch annehmbaren Träger in Berührung gebracht werden.

**20.** Verwendung nach Anspruch 18,
**dadurch gekennzeichnet, daß**
die krankheitserregenden Bakterien, *Chlamydia* oder Hefe mit 1 pmol Myeloperoxidase oder eosinophiler Peroxidase bis zu 200 pmol Myeloperoxidase oder eosinophiler Peroxidase pro ml in einem flüssigen, pharmazeutisch annehmbaren Träger in Berührung gebracht werden.

**21.** Verwendung nach Anspruch 18,
**dadurch gekennzeichnet, daß**
die krankheitserregenden Bakterien, *Chlamydia* oder Hefe mit 1 pmol Myeloperoxidase oder eosinophiler Peroxidase bis zu 1 nmol Myeloperoxidase oder eosinophiler Peroxidase pro Gramm in einem nichtflüssigen, pharmazeutisch annehmbaren Träger in Berührung gebracht werden.

**22.** Verwendung nach Anspruch 18,
**dadurch gekennzeichnet, daß**
die krankheitserregenden Bakterien, *Chlamydia* oder Hefe mit 10 pmol Myeloperoxidase oder eosinophiler Peroxidase bis zu 500 pmol Myeloperoxidase oder eosinophiler Peroxidase pro Gramm in einem nichtflüssigen, pharmazeutisch annehmbaren Träger in Berührung gebracht werden.

**23.** Verwendung nach Anspruch 17,
**dadurch gekennzeichnet, daß**
die Oxidase so wirkt, daß sie bei Vorhandensein von Sperma von 1 nmol bis zu 1 $\mu$mol Peroxid pro ml pro Minute erzeugt.

**24.** Verwendung nach Anspruch 17,
**dadurch gekennzeichnet, daß**
die Oxidase so wirkt, daß sie bei Vorhandensein von Sperma von 10 nmol bis zu 500 nmol Peroxid pro ml pro Minute erzeugt.

**25.** Verwendung nach Anspruch 17,
**dadurch gekennzeichnet, daß**
die Oxidase aus der aus Cholinoxidase, Cholesterinoxidase und Laktatoxidase bestehenden Gruppe ausgewählt wird.

**26.** Verwendung nach Anspruch 25,
**dadurch gekennzeichnet, daß**
die krankheitserregenden Bakterien, *Chlamydia* oder Hefe mit 1 Millieinheit bis 2 Einheiten Cholinoxidase oder Cholesterinoxidase pro ml in einem flüssigen, pharmazeutisch annehmbaren Träger in Berührung gebracht werden.

**27.** Verwendung nach Anspruch 25,
**dadurch gekennzeichnet, daß**
die krankheitserregenden Bakterien, *Chlamydia* oder Hefe mit 1 Millieinheit bis 2 Einheiten Cholinoxidase oder Cholesterinoxidase pro Gramm in einem nichtflüssigen, pharmazeutisch annehmbaren Träger in Berührung gebracht werden.

**28.** Verwendung nach Anspruch 25,
**dadurch gekennzeichnet, daß**
die krankheitserregenden Bakterien, *Chlamydia* oder Hefe mit 10 Millieinheiten bis 500 Millieinheiten Cholinoxidase oder Cholesterinoxidase pro ml in einem flüssigen, pharmazeutisch annehmbaren Träger in Berührung gebracht werden.

**29.** Verwendung nach Anspruch 25,

**dadurch gekennzeichnet, daß**

die krankheitserregenden Bakterien, *Chlamydia* oder Hefe mit 10 Millieinheiten bis 500 Millieinheiten Cholinoxidase oder Cholesterinoxidase pro Gramm in einem nichtflüssigen, pharmazeutisch annehmbaren Träger in Berührung gebracht werden.

**30.** Verwendung nach Anspruch 17,
**dadurch gekennzeichnet, daß**
die krankheitserregenden Bakterien, *Chlamydia* oder Hefe mit der Haloperoxidase und der Oxidase in einer Salbe, Gallerte, Schaum, Spülung oder einem Element zum Einführen in die Scheide in Berührung gebracht werden.


**Revendications**

**1.** Composition pharmaceutique pour inhiber la croissance, dans le vagin d'une femelle hôte, de bactéries pathogènes, *Chlamydia* ou levure entraînant une maladie sexuellement transmissible, comprenant des quantités d'une haloperoxydase et d'une oxydase choisies dans le groupe comprenant la choline oxydase, la cholestérol oxydase, la 1-aminoacide-oxydase, la polyamine oxydase, l'urate oxydase, la pyruvate oxydase et la lactate oxydase qui sont efficaces en présence de sperme ou de semence, pour inhiber la croissance des bactéries pathogènes, *Chlamydia* ou levure, avec un porteur pharmaceutiquement acceptable.

**2.** Composition de la revendication 1 dans laquelle l'haloperoxydase est choisie dans le groupe comprenant la myéloperoxydase et la peroxydase éosinophile.

**3.** Composition de la revendication 2 qui comprend de 0,1 pmol de myéloperoxydase ou de peroxydase éosinophile à 500 pmol de myéloperoxydase ou de peroxydase éosinophile par ml dans un porteur liquide pharmaceutiquement acceptable.

**4.** Composition de la revendication 2 qui comprend de 1 pmol de myéloperoxydase ou de peroxydase éosinophile à 1 nmol de myéloperoxydase ou de peroxydase éosinophile par gramme dans un porteur non liquide pharmaceutiquement acceptable.

**5.** Composition de la revendication 2 qui comprend de 1 pmol de myéloperoxydase ou de peroxydase éosinophile à 200 pmol de myéloperoxydase ou de peroxydase éosinophile par ml dans un porteur liquide pharmaceutiquement acceptable.

**6.** Composition de la revendication 2 qui comprend de 10 pmol de myéloperoxydase ou de peroxydase éosinophile à 500 pmol de myéloperoxydase ou de peroxydase éosinophile par gramme dans un porteur non liquide pharmaceutiquement acceptable.

**7.** Composition de la revendication 1 qui comprend de 1 milliunité à 2 unités d'oxydase spécifique de substrat de sperme par ml dans un porteur liquide pharmaceutiquement acceptable.

**8.** Composition de la revendication 1 qui comprend de 1 milliunité d'oxydase spécifique de substrat de sperme à 2 unités d'oxydase spécifique de substrat de sperme par gramme dans un porteur non liquide pharmaceutiquement acceptable.

**9.** Composition de la revendication 1 dans laquelle l'oxydase spécifique de substrat de sperme est choisie dans le groupe comprenant la choline oxydase, la cholestérol oxydase et la lactate oxydase.

**10.** Composition de la revendication 9 qui comprend de 1 milliunité à 2 unités de choline oxydase, de cholestérol oxydase par ml dans un porteur liquide pharmaceutiquement acceptable.

**11.** Composition de la revendication 9 comprenant de 1 milliunité à 2 unités de choline oxydase, de cholestérol oxydase par gramme dans un porteur non liquide pharmaceutiquement acceptable.

**12.** Composition de la revendication 9 qui comprend 10 milliunités à 500 milliunités de choline oxydase, de cholestérol oxydase par ml dans un porteur liquide pharmaceutiquement acceptable.

**13.** Composition de la revendication 9 qui comprend 10 milliunités à 500 milliunités de choline oxydase, de cholestérol oxydase par gramme dans un porteur non liquide pharmaceutiquement acceptable.

**14.** Composition de la revendication 1 dans laquelle le porteur pharmaceutiquement acceptable est sous forme d'un onguent, d'une gelée, d'une mousse, d'un lavage ou d'un comprimé vaginal.

**15.** Composition de la revendication 1 dans laquelle l'haloperoxydase est liée par liaison covalente à l'oxydase.

**16.** Composition de la revendication 1 dans laquelle l'haloperoxydase est liée par liaison non covalente à l'oxydase.

**17.** Utilisation d'une composition d'une haloperoxydase et d'une oxydase choisies dans le groupe comprenant la choline oxydase, la cholestérol oxydase, la 1-aminoacide-oxydase, la polyamine oxydase, l'urate oxydase, la pyruvate oxydase et la lactate oxydase dans un porteur pharmaceutiquement acceptable pour la fabrication d'un médicament efficace en présence de liquide séminal ou de sperme pour inhiber la croissance de bactéries pathogènes, *Chlamydia* ou levure.

**18.** Utilisation de la revendication 17 dans laquelle l'haloperoxydase est choisie dans le groupe comprenant la myéloperoxydase et la peroxydase éosinophile.

**19.** Utilisation de la revendication 18 dans laquelle la bactérie pathogène, *Chlamydia* ou levure sont mises en contact avec de 0,1 pmol de myéloperoxydase ou de peroxydase éosinophile à 500 pmol de myéloperoxydase ou de peroxydase éosinophile par ml dans un porteur liquide pharmaceutiquement acceptable.

**20.** Utilisation de la revendication 18 dans laquelle la bactérie pathogène, *Chlamydia* ou levure sont mises en contact avec de 1 pmol de myéloperoxydase ou de peroxydase éosinophile à 200 pmol de myéloperoxydase ou de peroxydase éosinophile par ml dans un porteur liquide pharmaceutiquement acceptable.

**21.** Utilisation de la revendication 18 dans laquelle la bactérie pathogène, *Chlamydia* ou levure sont mises en contact avec de 1 pmol de myéloperoxydase ou de peroxydase éosinophile à 1 nmol de myéloperoxydase ou de peroxydase éosinophile par gramme dans un porteur non liquide pharmaceutiquement acceptable.

**22.** Utilisation de la revendication 18 dans laquelle la bactérie pathogène, *Chlamydia* ou levure sont mises en contact avec de 10 pmol de myéloperoxydase ou de peroxydase éosinophile à 500 pmol de myéloperoxydase ou de peroxydase éosinophile par gramme dans un porteur non liquide pharmaceutiquement acceptable.

**23.** Utilisation de la revendication 17 dans laquelle l'oxydase est efficace pour générer de 1 nmol à 1 $\mu$mol de peroxyde par ml par minute en présence de semence.

**24.** Utilisation de la revendication 17 dans laquelle l'oxydase est efficace pour générer de 10 nmol à 500 nmol de peroxyde par ml par minute en présence de semence.

**25.** Utilisation de la revendication 17 dans laquelle l'oxydase est choisie dans le groupe comprenant la choline oxydase, la cholestérol oxydase et la lactate oxydase.

**26.** Utilisation de la revendication 25 dans laquelle la bactérie pathogène, *Chlamydia* ou levure sont mises en contact avec de 1 milliunité à 2 unités de choline oxydase ou de cholestérol oxydase par ml dans un porteur liquide pharmaceutiquement acceptable.

**27.** Utilisation de la revendication 25 dans laquelle la bactérie pathogène, *Chlamydia* ou levure sont mises en contact avec de 1 milliunité à 2 unités de choline oxydase ou de cholestérol oxydase par gramme dans un porteur non liquide pharmaceutiquement acceptable.

**28.** Utilisation de la revendication 25 dans laquelle la bactérie pathogène, *Chlamydia* ou levure sont mises en contact avec de 10 milliunités à 500 milliunités de choline oxydase ou de cholestérol oxydase par ml dans un porteur liquide pharmaceutiquement acceptable.

**29.** Utilisation de la revendication 25 dans laquelle la bactérie pathogène, *Chlamydia* ou levure sont mises en contact avec de 10 milliunités à 500 milliunités de choline oxydase ou de cholestérol oxydase par gramme dans un porteur non liquide pharmaceutiquement acceptable.

30. Utilisation de la revendication 17 dans laquelle la bactérie pathogène, *Chlamydia* ou levure sont mises en contact avec l'haloperoxydase et l'oxydase dans un onguent, une gelée, une mousse, un lavage ou un comprimé vaginal acceptables.